# EUROPÄISCHE PATENTANMELDUNG

(11) **EP 2 668 964 A1**
(43) Veröffentlichungstag der Anmeldung: **04.12.2013**
(21) Anmeldenummer: 13160339.1
(22) Anmeldetag: 21.03.2013
(51) Int. Cl.: A61L 9/015, A61L 9/20, B60Q 3/00

(54) **Reinigungseinrichtung und Verfahren zum Reinigen von Fahrzeuginnenräumen**

(30) Priorität: 04.04.2012 DE 102012006972
(71) Anmelder: SBF Spezialleuchten GmbH, 04316 Leipzig (DE)
(72) Erfinder: Ilmer, Michael, 04316 Leipzig (DE)
(74) Vertreter: Söltenfuss, Dirk Christian

(57) **Zusammenfassung**

Eine Reinigungseinrichtung zum automatischen oder halbautomatischen Reinigen von Fahrzeuginnenräumen zum Beispiel eines Schienenfahrzeuges weist wenigstens eine Strahlungsvorrichtung (10), welche Strahlung in wenigstens einem vorbestimmten Wellenlängenbereich zwischen etwa 150 nm und etwa 350 nm emittiert, und eine Befestigungseinrichtung (12) zum Montieren der wenigstens einen Strahlungsvorrichtung (10) in einem Fahrzeuginnenraum derart, dass die von der wenigstens einen Strahlungsvorrichtung (10) emittierte Strahlung im Wesentlichen auf wenigstens einen Einrichtungsgegenstand (14) des Fahrzeuginnenraums und/oder in den Fahrzeuginnenraum (22) gerichtet ist, um den Einrichtungsgegenstand (14) bzw. die Luft im Fahrzeuginnenraum (22) zu desinfizieren, auf.

## Beschreibung

Die Erfindung betrifft eine Reinigungseinrichtung und ein Verfahren zum Reinigen von Fahrzeuginnenräumen, insbesondere von Schienenfahrzeugen.

Speziell in (öffentlichen) Transportmitteln zum Befördern einer größeren Anzahl von Passagieren, wie Zügen, Flugzeugen, Schiffen, Omnibussen und dergleichen, gibt es zahlreiche Gegenstände, die regelmäßig in Kontakt mit vielen verschiedenen Personen kommen. Es ist notwendig, diese Gegenstände regelmäßig und gründlich von Bakterien und Keimen zu reinigen. Dies geschieht üblicherweise im Wesentlichen manuell in einem mehrstufigen System (Zwischenreinigungen während der Fahrt, Tagesreinigungen, Grund- und Spezialreinigungen). Ein weiteres Problem in solchen Transportmitteln sind die Gerüche in den Fahrzeuginnenräumen, die von den Passagieren als unangenehm empfunden werden und mit herkömmlichen Reinigungssystemen kaum zu entfernen sind.

Es besteht daher Bedarf an einem zuverlässigen, gründlichen und möglichst einfachen Reinigungssystem für solche Transportmittel.

In diesem Zusammenhang ist auch die keimtötende Wirkung von UV-Strahlung allgemein bekannt. Speziell in der Fahrzeugtechnik wird dieses Reinigungsverfahren bereits zum Beispiel zur Desinfektion von Klimaanlagen eingesetzt (vgl. z.B. DE 202 14 699 U1 und DE 10 2009 015 088 A1).

Ferner beschreibt die DE 20 2011 050 141 U1 eine Dunstabzugshaube, die mit einer Behandlungseinrichtung zum zumindest teilweisen Entfernen unerwünschter Stoffe und Gerüche aus der Abluft ausgestattet ist. Die Behandlungseinrichtung weist eine UV-Lampe zur Ozonbildung und einen nachgeschalteten Aktivkohlefilter auf.

Es ist die Aufgabe der Erfindung, eine verbesserte Reinigungseinrichtung zum Reinigen von Fahrzeuginnenräumen zu schaffen, mit welcher bei möglichst geringem Aufwand eine gründliche Reinigungswirkung für Fahrzeuginnenräume erzielt werden kann.

Diese Aufgabe wird gelöst durch eine Reinigungseinrichtung zum Reinigen von Fahrzeuginnenräumen mit den Merkmalen des Anspruches 1. Besonders bevorzugte Weiterbildungen und Ausgestaltungen der Erfindung sind in den abhängigen Ansprüchen angegeben.

Die erfindungsgemäße Reinigungseinrichtung zum Reinigen von Fahrzeuginnenräumen weist wenigstens eine Strahlungsvorrichtung, welche Strahlung in wenigstens einem vorbestimmten Wellenlängenbereich zwischen etwa 150 nm und etwa 350 nm emittiert, und eine Befestigungseinrichtung zum Montieren der wenigstens einen Strahlungsvorrichtung in einem Fahrzeuginnenraum derart, dass die von der wenigstens einen Strahlungsvorrichtung emittierte Strahlung im Wesentlichen auf wenigstens einen Einrichtungsgegenstand des Fahrzeuginnenraums und/oder in den Fahrzeuginnenraum gerichtet ist, um den Einrichtungsgegenstand und/oder die Luft im Fahrzeuginnenraum zu desinfizieren, auf.

Erfindungsgemäß wird vorgeschlagen, eine Strahlungsvorrichtung in einem Fahrzeuginnenraum zu montieren, um Einrichtungsgegenstände des Fahrzeuginnenraums und/oder die Luft im Fahrzeuginnenraum mittels UV-Bestrahlung zu desinfizieren.

Durch die Montage der entsprechenden Strahlungsvorrichtung(en) im Fahrzeuginnenraum ist eine automatische oder zumindest halbautomatische Reinigungsprozedur des Fahrzeuginnenraums bzw. seiner Einrichtungsgegenstände möglich. Der Reinigungsaufwand kann im Vergleich zu herkömmlichen manuellen Reinigungsvorgängen reduziert werden. Durch die (Halb-)Automatik des Reinigungsvorganges kann zudem die Zuverlässigkeit der Reinigung bzw. Desinfektion erhöht werden; dies insbesondere auch deshalb, weil die Strahlung in vordefinierter Weise (Richtung, Dosis, etc.) auf die betreffenden Einrichtungsgegenstände bzw. in den Fahrzeuginnenraum gerichtet werden kann. Weiter kann der Desinfektionsvorgang mit der erfindungsgemäßen Reinigungseinrichtung auch selbsttätig über Nacht durchgeführt werden, sodass problemlos die für eine effektive Desinfektion erforderlichen Bestrahlungsdosen erreicht werden können.

Die erfindungsgemäße Desinfektion basiert auf zwei Funktionsprinzipien. Einerseits kann eine Desinfektion mittels UV-Bestrahlung im Wellenlängenbereich zwischen etwa 200 nm und etwa 350 (Hauptwellenlängen vorzugsweise etwa 253,7 nm, 245 nm und 265 nm) erfolgen. Diese UV-Bestrahlung inaktiviert zum Beispiel Mikroorganismen und dergleichen. Andererseits kann in der Luft mittels UV-Bestrahlung im Wellenlängenbereich zwischen etwa 150 nm und etwa 200 nm (Hauptwellenlänge vorzugsweise etwa 185 nm) aus Sauerstoff durch Spaltung der Sauerstoffmoleküle O₂ Ozon O₃ gebildet werden. Dieses Ozon O₃ wird dann durch die UV-Linie bei 245 nm zersetzt, untre Bildung hochreaktiver, freier Sauerstoffradikale O*, welche dann - zum Teil über die Bildung weiterer Radikale - dann die eigentliche Desinfektion bewirken. Diese (Sauerstoff-)Radikale töten zum Beispiel Keime und Bakterien ab und oxidieren bzw. spalten chemische Verbindungen zu unschädlichen Verbindungen und Kohlendioxid.

Unter dem Begriff "Fahrzeuginnenraum" werden in diesem Zusammenhang grundsätzlich alle Arten von Innenräumen von Fahrzeugen, wie Schienenfahrzeugen, Flugzeugen, Schiffen, Omnibussen und dergleichen, verstanden. Vorzugsweise handelt es sich bei dem Fahrzeuginnenraum um einen Innenraum des jeweiligen Fahrzeuges, der von Personen, insbesondere auch von Passagieren genutzt wird. Hierzu zählen insbesondere Passagierkabinen (Großraum, Abteil, Schlafabteil, etc.), Toiletten, Waschräume, Wickelräume und dergleichen.

Der Begriff "Einrichtungsgegenstand" des Fahrzeuginnenraums soll in diesem Zusammenhang jede Art von Einrichtungsgegenstand in dem jeweiligen Fahrzeuginnenraum umfassen. Vorzugsweise handelt es sich bei diesen Einrichtungsgegenständen, welche von Personen bzw. Passagieren benutzt oder berührt werden. Hierzu zählen grundsätzlich Einrichtungsgegenstände mit beliebigen Oberflächen, einschließlich Textil, Metall, Holz, Kunststoff und dergleichen. Zu den Einrichtungsgegenständen, die mit Hilfe der erfindungsgemäßen Reinigungseinrichtung desinfiziert werden sollen, zählen vorzugsweise Sitze, Kopfstützen, Armlehnen, Liegen, Tische, Handläufe, Türgriffe, Haltegriffe, Toiletteninventar, Fußböden und dergleichen, ohne dass die Erfindung auf diese Anwendungen beschränkt sein soll.

Unter einer "Strahlungsvorrichtung" ist jede Art von Vorrichtung mit wenigstens einer Strahlungsquelle zu verstehen. Erfindungsgemäß soll die Strahlungsvorrichtung Strahlung in wenigstens einem vorbestimmten Wellenlängenbereich zwischen etwa 150 nm und etwa 350 nm emittieren. Hierbei handelt es sich allgemein um UV-Strahlung, vorzugsweise um UV-C-Strahlung. Die Strahlungsvorrichtung ist ausgebildet, um UV-Strahlung in einem Wellenlängenbereich oder in zwei, drei oder mehr Wellenlängenbereichen zu emittieren. Im Fall von zwei oder mehr Wellenlängenbereichen können diese getrennt sein oder teilweise überlappen. Die vorbestimmten Wellenlängenbereiche werden durch die Strahlungsvorrichtung eingestellt; dies geschieht vorzugsweise über die Strahlungsquellen selbst, entsprechende Filter und/oder geeignete Optiken (z.B. Quarzgläser, etc.).

Die Strahlungsvorrichtung im Sinne der Erfindung enthält eine oder mehrere Strahlungsquellen. Im Fall mehrerer Strahlungsquellen können diese insbesondere in Bezug auf die Wellenlängenbereiche der emittierten Strahlung gleichartig oder unterschiedlich sein. Die Strahlungsvorrichtung bzw. deren Strahlungsquelle(n) emittiert die UV-Strahlung vorzugsweise ununterbrochen, intermittierend oder gepulst. Zudem weist die Strahlungsvorrichtung im Sinne der Erfindung bevorzugt ein Gehäuse und eine der wenigstens einen Strahlungsquelle zugeordnete Optik auf. Die Strahlungsvorrichtung enthält vorzugsweise UV-LEDs, Quecksilber-Niederdruckstrahler oder Mitteldruckstrahler und/oder Xenon-Excimerstrahler.

Im Sinne der Erfindung dient die "Befestigungseinrichtung" allgemein dem Montieren der wenigstens einen Strahlungsvorrichtung in einem Fahrzeuginnenraum. Die Montage kann dabei lösbar oder unlösbar ausgestaltet sein. Außerdem kann die Montage im Fahrzeuginnenraum stationär oder bewegbar ausgestaltet sein. Ferner kann die Strahlungsvorrichtung in der Befestigungseinrichtung fest oder bewegbar angeordnet sein.

Vorzugsweise weist die Reinigungseinrichtung eine Vielzahl von Strahlungsvorrichtungen mit zugehörigen Befestigungseinrichtung(en) im jeweiligen Fahrzeuginnenraum auf, die entsprechend den zu desinfizierenden Einrichtungsgegenständen im Fahrzeuginnenraum verteilt und angeordnet sind.

Die erfindungsgemäße Reinigungseinrichtung weist vorzugsweise eine Steuervorrichtung auf, welche den Betrieb der Strahlungsvorrichtung(en) steuert, um eine effektive Desinfektion der Einrichtungsgegenstände im Fahrzeuginnenraum zu erzielen. Die Steuervorrichtung kann vorzugsweise die Bestrahlungsstärke und/oder die Bestrahlungsdauer und/oder die Strahlungsfokussierung und damit die Dosierung der UV-Bestrahlung steuern. Ferner kann die Steuervorrichtung ggf. die vorbestimmten Wellenlängenbereiche auswählen. Außerdem kann die Steuervorrichtung ggf. eine Bewegung der Strahlungsvorrichtung(en) in der Befestigungseinrichtung und/oder eine Bewegung von einzelnen Strahlungsquellen der Strahlungsvorrichtung(en) steuern. Weiter kann die Steuervorrichtung vorzugsweise die Reinigungszyklen steuern; bevorzugt werden Toiletten nach jeder Benutzung desinfiziert, während bei anderen Passagierkabinen und deren Einrichtungsgegenständen eine Tagesreinigung ausreichend sein kann.

In einer bevorzugten Ausgestaltung der Erfindung ist die wenigstens eine Strahlungsvorrichtung ausgebildet, um Strahlung in einem ersten vorbestimmten Wellenlängenbereich zwischen etwa 200 nm und etwa 350 nm und/oder Strahlung in einem zweiten vorbestimmten Wellenlängenbereich zwischen etwa 150 nm und etwa 220 nm zu emittieren.

Die Strahlungsvorrichtungen der erfindungsgemäßen Reinigungseinrichtung sind somit entweder zur Desinfektion mittels UV-Bestrahlung oder zur Desinfektion mittels Ozonbildung oder zur Desinfektion mittels beider Wirkmechanismen ausgebildet. Der erste vorbestimmte Wellenlängenbereich zur Desinfektion mittels UV-Bestrahlung umfasst Wellenlängen von wenigstens etwa 200 nm, bevorzugter wenigstens etwa 240 nm, und höchstens etwa 350 nm, bevorzugter höchstens etwa 270 nm, und am bevorzugtesten Wellenlängen um etwa 245 nm, 254 nm und 265 nm. Der zweite vorbestimmte Wellenlängenbereich zur Desinfektion mittels Ozonbildung umfasst Wellenlängen zwischen etwa 150 nm und etwa 220 nm, am bevorzugtesten um etwa 185 nm. Bei der Bestrahlung im ersten Wellenlängenbereich können insbesondere Bereiche gereinigt werden, welche direkt angestrahlt werden; bei der Bestrahlung im zweiten Wellenlängenbereich können durch das gebildete Ozon vorzugsweise auch nicht direkt anstrahlbare Bereiche (z.B. Ecken, Winkel, verdeckte Bereiche, etc.) gereinigt werden.

Bei dieser Ausgestaltung ist die wenigstens eine Strahlungsvorrichtung vorzugsweise ausgebildet ist, um die Strahlung im ersten vorbestimmten Wellenlängenbereich und die Strahlung im zweiten vorbestimmten Wellenlängenbereich einmal oder mehrmals nacheinander zu emittieren. Vorzugsweise emittiert die Strahlungsvorrichtung für einen Reinigungsvorgang zunächst Strahlung im zweiten Wellenlängenbereich und anschließend Strahlung im ersten Wellenlängenbereich, wobei diese Abfolge mehrfach wiederholt werden kann. Die UV-Strahlung im zweiten Wellenlängenbereich unterstützt dabei auch die Zersetzung des zuvor gebildeten Ozons in die Sauerstoffradikale.

Bei einer anderen bevorzugten Ausgestaltung der Erfindung weist die wenigstens eine Strahlungsvorrichtung wenigstens eine Strahlungsquelle auf, welche Strahlung in beiden vorbestimmten Wellenlängenbereichen gleichzeitig emittiert. Hierzu geeignete Strahlungsquellen sind beispielsweise Quecksilber-Niderdruckstrahler und Hochdruckstrahgler und Xenon-Excimerstrahler.

In einer weiteren bevorzugten Ausgestaltung der Erfindung ist ferner wenigstens ein Strahlungssensor vorgesehen, der derart ausgebildet ist und derart in dem Fahrzeuginnenraum angeordnet werden kann, dass er eine von der wenigstens einen Strahlungsvorrichtung emittierte Strahlung erfasst. Mit Hilfe des wenigstens einen Strahlungssensors kann vorzugsweise die Funktionsfähigkeit der wenigstens einen im Fahrzeuginnenraum montierten Strahlungsvorrichtung überwacht werden.

In einer weiteren bevorzugten Ausgestaltung der Erfindung ist ferner wenigstens ein Ozonsensor vorgesehen, der derart ausgebildet ist und derart in dem Fahrzeuginnenraum angeordnet werden kann, dass er einen Ozongehalt im Fahrzeuginnenraum erfasst. Der wenigstens eine Ozonsensor ist dabei geeignet, den Ozongehalt im Fahrzeuginnenraum direkt oder indirekt zu erfassen. Mit Hilfe des Ozonsensors kann sichergestellt werden, dass der Ozongehalt nach einem erfindungsgemäßen Reinigungsvorgang wieder ausreichend abgesunken ist, damit der Fahrzeuginnenraum von Personen wieder gefahrlos betreten werden kann.

In einer weiteren bevorzugten Ausgestaltung der Erfindung ist ferner wenigstens ein Personensensor vorgesehen, der derart ausgebildet ist und derart in dem Fahrzeuginnenraum angeordnet werden kann, dass er die Anwesenheit von Personen in dem Fahrzeuginnenraum erfasst. Mit Hilfe des wenigstens einen Personensensors kann vorzugsweise sichergestellt werden, dass die erfindungsgemäße Reinigungseinrichtung nur in einem leeren Fahrzeuginnenraum, in dem sich keine Personen aufhalten, in Betrieb genommen wird. Auf diese Weise kann bevorzugt eine Gesundheitsgefährdung von Personen durch eine UV-Strahlung der Reinigungseinrichtung vermieden werden.

In einer noch weiteren bevorzugten Ausgestaltung der Erfindung ist ferner wenigstens eine Antriebsvorrichtung zum Bewegen wenigstens einer der wenigstens einen Strahlungsvorrichtung entlang eines vorbestimmten Weges in dem Fahrzeuginnenraum vorgesehen. Je nach Ausführungsform wird vorzugsweise eine Strahlungsvorrichtung innerhalb einer Befestigungseinrichtung (z.B. in deren Schienenführung) bewegt oder wird eine Befestigungseinrichtung mit der daran angeordneten Strahlungsvorrichtung in dem Fahrzeuginnenraum bewegt. Durch eine Bewegung einer Strahlungsvorrichtung durch den Fahrzeuginnenraum kann die Anzahl der für die Desinfektion des gesamten Fahrzeuginnenraums benötigten Strahlungsvorrichtungen reduziert werden.

In einer Ausgestaltung der Erfindung ist wenigstens einer der wenigstens einen Strahlungsvorrichtung in eine Beleuchtungseinrichtung des Fahrzeuginnenraums integriert oder mit einer solchen kombiniert. Diese Ausführungsform ermöglicht eine platzsparende Montage der Reinigungseinrichtung zusammen mit dem Beleuchtungssystem des Fahrzeuginnenraums.

In einer Ausgestaltung der Erfindung weist die Reinigungseinrichtung ferner wenigstens eine Belüftungseinrichtung zum Belüften des Fahrzeuginnenraums auf oder ist mit einer solchen verbunden. Mit Hilfe der Belüftungseinrichtung können vorzugsweise Verunreinigungen der Raumluft, welche ggf. bei der UV-Bestrahlung der Einrichtungsgegenstände entstehen können, und/oder Reste des durch die UV-Strahlung gebildeten Ozons aus dem Fahrzeuginnenraum entfernt werden. Auf diese Weise kann eine Gesundheitsgefährdung von Personen und Passagieren, die nach einem Desinfektionsvorgang den Fahrzeuginnenraum betreten, reduziert werden.

In einer weiteren Ausgestaltung der Erfindung weist die Reinigungseinrichtung ferner wenigstens eine Reinigungsmittel-Ausbringeinrichtung zum Ausbringen eines Reinigungsmittels in den Fahrzeuginnenraum und/oder auf Einrichtungsgegenstände in dem Fahrzeuginnenraum auf oder ist mit einer solchen verbunden. Durch das Ausbringen von Reinigungsmitteln, zusätzlich zu der UV-Bestrahlung kann die Desinfektionswirkung der Reinigungseinrichtung weiter erhöht werden. Bei den Reinigungsmitteln handelt es sich vorzugsweise um chemische Reinigungsmittel, bevorzugt um oxidativ wirkende Reinigungsmittel, mit denen sich zusammen mit der UV-Bestrahlung ein Synergieeffekt hinsichtlich der Desinfektionswirkung erzielen lässt. Die Reinigungsmittel werden vorzugsweise flüssig bereitgestellt und auf die zu desinfizierenden Einrichtungsgegenstände versprüht.

Gegenstand der Erfindung ist auch ein Fahrzeug, insbesondere ein Schienenfahrzeug, mit wenigstens einer Reinigungseinrichtung gemäß der Erfindung.

Gegenstand der Erfindung ist schließlich auch ein Verfahren zum Reinigen von Fahrzeuginnenräumen, insbesondere von Schienenfahrzeugen, bei welchem Einrichtungsgegenstände des Fahrzeuginnenraums und/oder die Luft im Fahrzeuginnenraum mittels wenigstens einer in dem Fahrzeuginnenraum montierten Strahlungsvorrichtung mit einer Strahlung in wenigstens einem vorbestimmten Wellenlängenbereich zwischen etwa 150 nm und etwa 350 nm bestrahlt werden.

Vorzugsweise werden eine Bestrahlung in einem ersten vorbestimmten Wellenlängenbereich zwischen etwa 200 nm und etwa 350 nm und/oder eine Bestrahlung in einem zweiten vorbestimmten Wellenlängenbereich zwischen etwa 150 nm und etwa 200 nm durchgeführt.

Vorzugsweise wird zur Ausführung dieses Reinigungsverfahrens eine oben erläuterte Reinigungseinrichtung gemäß der Erfindung verwendet.

Zum Zwecke einer ausreichenden Desinfektion werden die Einrichtungsgegenstände des Fahrzeuginnenraums vorzugsweise mit einer Dosis von wenigstens etwa 5,0 mJ/cm², bevorzugter von wenigstens etwa 10,0 mJ/cm², am bevorzugtesten von über etwa 40 mJ/cm² bestrahlt.

Die mit diesem Reinigungsverfahren erzielbaren Vorteile und die zugehörigen Begriffsdefinitionen und bevorzugten Ausgestaltungen entsprechen jenen, die oben in Zusammenhang mit der erfindungsgemäßen Reinigungseinrichtung beschrieben worden sind.

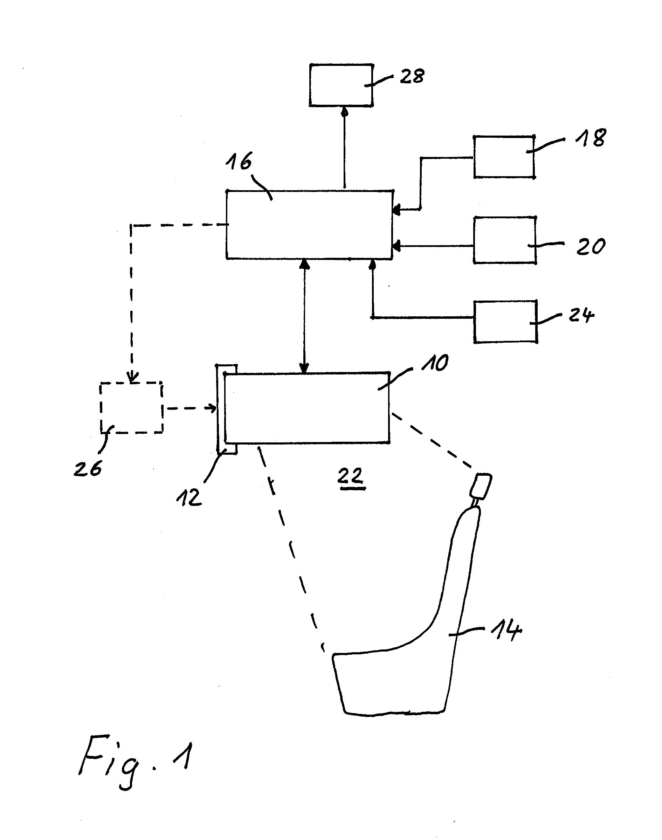

Obige sowie weitere Merkmale und Vorteile der Erfindung werden aus der nachfolgenden Beschreibung eines bevorzugten, nicht-einschränkenden Ausführungsbeispiels anhand der beiliegenden Zeichnungen besser verständlich. Darin zeigt die einzige Figur 1 ein schematisches Blockschaltbild einer Reinigungseinrichtung gemäß einem Ausführungsbeispiel der Erfindung.

Die Erfindung wird nachfolgend am Beispiel einer Reinigungseinrichtung für ein Schienenfahrzeug näher erläutert, ohne dass die Erfindung auf diese spezielle Anwendung beschränkt sein soll.

In Figur 1 ist beispielhaft eine Strahlungsvorrichtung 10 gezeigt, die mittels einer zugehörigen Befestigungseinrichtung 12 in einem Fahrzeuginnenraum montiert werden kann. Die Reinigungseinrichtung der Erfindung ist aber üblicherweise mit einer größeren Anzahl von Strahlungsvorrichtungen 10 versehen, die mittels mehrerer oder gemeinsamer Befestigungseinrichtungen 12 im Fahrzeuginnenraum montierbar sind, und deren Anzahl und Verteilung an den jeweiligen Fahrzeuginnenraum bzw. seine Einrichtungsgegenstände angepasst sind. In Figur 1 ist als ein möglicher Einrichtungsgegenstand 14 ein Sitz mit einer Kopfstütze dargestellt sowie der Bereich des Fahrzeuginnenraums 22 oberhalb des Sitzes 14 angedeutet.

Die Strahlungsvorrichtung 10 enthält eine oder mehrere Strahlungsquellen, zum Beispiel in Form von Leuchtdioden, welche UV-Strahlung, insbesondere UV-C-Strahlung emittieren. Eine erste Anzahl von Strahlungsquellen der Strahlungsvorrichtung 10 emittiert vorzugsweise Strahlung in einem ersten vorbestimmten Bereich zwischen etwa 200 nm und etwa 350 nm, bevorzugter zwischen etwa 240 nm und etwa 270 nm. Durch Bestrahlung in diesem Wellenlängenbereich können pathogene Erreger, Keime, Bakterien, Viren und dergleichen effektiv abgetötet bzw. unschädlich gemacht werden. Eine zweite Anzahl von Strahlungsquellen der Strahlungsvorrichtung 10 emittiert vorzugsweise Strahlung in einem zweiten vorbestimmten Bereich zwischen etwa 150 nm und etwa 220 nm. Durch Bestrahlung in diesem Wellenlängenbereich wird aus Luftsauerstoff Ozon gebildet, welches in Sauerstoffradikale zerfällt, durch welche Keime, Bakterien und dergleichen effektiv abgetötet bzw. unschädlich gemacht werden und Gerüche neutralisiert werden. Anstelle unterschiedlicher Strahlungsquellen können zum Beispiel auch gleichartige Strahlungsquellen mit einem Wellenlängenbereich zwischen etwa 150 nm und etwa 350 nm in Kombination mit unterschiedlichen Filtern und/oder Gehäuseoptiken eingesetzt werden. Außerdem können zum Beispiel Quecksilber-Niederdurckstrahler, Quecksilber-Mitteldruckstrahler oder Xenon-Excimerstrahler eingesetzt werden, welche UV-Licht gleichzeitig in beiden Wellenlängenbereichen emittieren, welche für die beiden hier interessierenden Reinigungseffekte verantwortlich sind.

Die Strahlungsvorrichtung(en) 10 ist/sind mit einer Steuervorrichtung 16 verbunden. Die Steuervorrichtung 16 steuert den Betrieb der Strahlungsvorrichtung(en) 10 bzw. deren Strahlungsquellen entsprechend der gewünschten Desinfektion der jeweiligen Einrichtungsgegenstände 14. Insbesondere steuert die Steuervorrichtung 16 die Bestrahlungsdosis, indem sie die Dauer, die Wellenlänge, die Richtung und/oder die Fokussierung der Bestrahlung durch die Strahlungsquellen bzw. Strahlungsquellengruppensteuert, je nachdem welche Parameter in der Strahlungsvorrichtung 10 einstellbar sind. Außerdem kann die Steuervorrichtung 16 eine Abfolge von möglichen unterschiedlichen UV-Bestrahlungen steuern.

Die Strahlungsvorrichtung 10 emittiert die UV-Strahlung wahlweise bzw. je nach Ausführungsform und Anwendungsfall ununterbrochen, intermittierend oder gepulst. Gegebenenfalls kann die Steuervorrichtung 16 auch den Betriebsmodus der Strahlungsvorrichtung 10 bzw. deren Strahlungsquellen variabel einstellen oder die Pulsdauer und/oder die Pulsfrequenz steuern.

Wie in Figur 1 veranschaulicht, ist die Steuervorrichtung 16 zudem mit wenigstens einem Strahlungssensor 18, wenigstens einem Ozonsensor 24 und wenigstens einem Personensensor 20 verbunden.

Der Strahlungssensor 18 ist so ausgelegt und in dem Fahrzeuginnenraum positioniert, dass er die von einer Strahlungsvorrichtung emittierte UV-Strahlung erfassen kann. Mit Hilfe des Strahlungssensors 18 kann die Steuervorrichtung 16 die Funktionsfähigkeit der jeweiligen Strahlungsvorrichtung 10 überwachen und ggf. eine entsprechende Fehlermeldung ausgeben.

Der Ozonsensor 18 ist so ausgelegt und in dem Fahrzeuginnenraum positioniert, dass er den Ozongehalt im Fahrzeuginnenraum direkt oder indirekt erfassen kann. Mit Hilfe des Ozonsensors 18 kann erkannt werden, ob der jeweilige Fahrzeuginnenraum nach einem Reinigungsvorgang wieder gefahrlos betreten werden kann.

Der Personensensor 20 soll die Anwesenheit von Personen in dem jeweiligen Fahrzeuginnenraum überwachen. Der Personensensor 20 kann zu diesem Zweck zum Beispiel einen Bewegungsmelder, einen Infrarot-Sensor, eine Kamera und dergleichen enthalten. Üblicherweise sind in einem Fahrzeuginnenraum - je nach Größe und Gestaltung - mehrere Personensensoren 20 verteilt angeordnet. Die Steuervorrichtung 16 kann mit Hilfe des Personensensors 20 zum Beispiel sicherstellen, dass die Reinigungseinrichtung bzw. deren Strahlungsvorrichtung(en) 10 in dem jeweiligen Fahrzeuginnenraum nur dann in Betrieb genommen werden, d.h. eine UV-Strahlungsemission erfolgt, wenn sich keine Personen in diesem Fahrzeuginnenraum befinden. Auf diese Weise kann eine Gesundheitsgefährdung von Personen (Passagiere, Zugpersonal, Reinigungsdienst, usw.) durch UV-Strahlung und Ozon verhindert werden.

Mit der beschriebenen Reinigungseinrichtung kann eine automatische oder zumindest halbautomatische Reinigung bzw. Desinfektion der Einrichtungsgegenstände in dem Fahrzeuginnenraum durchgeführt werden. Die Strahlungsvorrichtungen 10 können zu diesem Zweck fest in dem Fahrzeuginnenraum installiert sein oder jeweils bei Bedarf (lösbar) montiert werden. Außerdem werden die Strahlungsvorrichtungen 10 über die Steuervorrichtung 16 zentral angesteuert.

Je nach Gestaltung und Ausstattung des Fahrzeuginnenraums können die Strahlungsvorrichtungen 10 auch bewegbar in dem Fahrzeuginnenraum montiert werden. Zum Beispiel können sie in geeignete Führungsschienen als Befestigungseinrichtung 12 eingehängt werden. Die Bewegung der Strahlungsvorrichtungen 10 erfolgt dann mittels einer Antriebsvorrichtung 22 entlang eines vorbestimmten Weges und in einem vorbestimmten zeitlichen Bewegungsprofil (kontinuierliche oder schrittweise Bewegung, etc.). Die Antriebsvorrichtung 22 wird in diesem Fall bevorzugt ebenfalls von der Steuervorrichtung 16 angesteuert.

Obwohl zum Teil nicht dargestellt, kann die Reinigungseinrichtung mit weiteren Einrichtungen gekoppelt sein bzw. solche aufweisen.

Hierzu zählt zum Beispiel eine Belüftungseinrichtung 28, um den Fahrzeuginnenraum während und/oder nach einem Reinigungsvorgang durch UV-Bestrahlung zu belüften. Etwaige Verunreinigungen der Raumluft, welche durch oder während der UV-Bestrahlung der Einrichtungsgegenstände entstehen, können so aus dem Fahrzeuginnenraum entfernt werden. Ebenso können etwaige Reste des durch die UV-Bestrahlung gebildeten Ozons aus dem Fahrzeuginnenraum entfernt werden. Eine mögliche Gesundheitsgefährdung von Personen kann auf diese Weise reduziert werden. Der Betrieb der Belüftungseinrichtung 28 erfolgt vorzugsweise automatisch und ist an den Betrieb der Strahlungsvorrichtungen 10 gekoppelt. Die Steuervorrichtung 16 steuert bevorzugt auch diese Belüftungseinrichtung 28.

Zu den weiteren (optionalen) Einrichtungen der erfindungsgemäßen Reinigungseinrichtung zählt auch eine Reinigungsmittel-Ausbringeinrichtung. Diese Reinigungsmittel-Ausbringeinrichtung dient dem Ausbringen eines (zusätzlichen) Reinigungsmittels in den Fahrzeuginnenraum bzw. auf die Einrichtungsgegenstände 14 in dem Fahrzeuginnenraum. Durch das Ausbringen von Reinigungsmitteln vor, nach oder gleichzeitig zur UV-Bestrahlung kann die Desinfektionswirkung der erfindungsgemäßen Reinigungseinrichtung weiter erhöht werden. Der Betrieb der Reinigungsmittel-Ausbringeinrichtung erfolgt vorzugsweise automatisch und ist an den Betrieb der Strahlungsvorrichtungen 10 gekoppelt. Die Steuervorrichtung 16 steuert bevorzugt auch diese Ausbringeinrichtung.

Bei den Reinigungsmitteln handelt es sich beispielsweise um chemische Reinigungsmittel, bevorzugt um oxidativ wirkende Reinigungsmittel, mit denen sich zusammen mit der UV-Bestrahlung ein Synergieeffekt hinsichtlich der Desinfektionswirkung erzielen lässt. Die Reinigungsmittel werden zum Beispiel auf die zu desinfizierenden Einrichtungsgegenstände 14 versprüht bzw. in Form eines Sprühnebels oder eines Aerosols aufgebracht.

Alternativ kann das Ausbringen von zusätzlichen Reinigungsmitteln auf die zu desinfizierenden Einrichtungsgegenstände 14 vor der Anwendung der Reinigungseinrichtung, d.h. der UV-Bestrahlung auch manuell durch das Reinigungspersonal erfolgen.

## Patentansprüche

1. Reinigungseinrichtung zum Reinigen von Fahrzeuginnenräumen, insbesondere von Schienenfahrzeugen, mit
wenigstens einer Strahlungsvorrichtung (10), welche Strahlung in wenigstens einem vorbestimmten Wellenlängenbereich zwischen etwa 150 nm und etwa 350 nm emittiert;
einer Befestigungseinrichtung (12) zum Montieren der wenigstens einen Strahlungsvorrichtung (10) in einem Fahrzeuginnenraum derart, dass die von der wenigstens einen Strahlungsvorrichtung (10) emittierte Strahlung im Wesentlichen auf wenigstens einen Einrichtungsgegenstand (14) des Fahrzeuginnenraums und/oder in den Fahrzeuginnenraum (22) gerichtet ist, um den Einrichtungsgegenstand (14) bzw. die Luft im Fahrzeuginnenraum (22) zu desinfizieren.

2. Reinigungseinrichtung nach Anspruch 1,
**dadurch gekennzeichnet, dass**
die wenigstens eine Strahlungsvorrichtung (10) ausgebildet ist, um Strahlung in einem ersten vorbestimmten Wellenlängenbereich zwischen etwa 200 nm und etwa 350 nm und/oder Strahlung in einem zweiten vorbestimmten Wellenlängenbereich zwischen etwa 150 nm und etwa 220 nm zu emittieren.

3. Reinigungseinrichtung nach Anspruch 2,
**dadurch gekennzeichnet, dass**
die wenigstens eine Strahlungsvorrichtung (10) ausgebildet ist, um die Strahlung im ersten vorbestimmten Wellenlängenbereich und die Strahlung im zweiten vorbestimmten Wellenlängenbereich einmal oder mehrmals nacheinander oder gleichzeitig zu emittieren.

4. Reinigungseinrichtung nach einem der vorhergehenden Ansprüche,
**dadurch gekennzeichnet, dass**
wenigstens ein Strahlungssensor (18) vorgesehen ist, der derart ausgebildet ist und derart in dem Fahrzeuginnenraum angeordnet werden kann, dass er eine von der wenigstens einen Strahlungsvorrichtung (10) emittierte Strahlung erfasst.

5. Reinigungseinrichtung nach einem der vorhergehenden Ansprüche,
**dadurch gekennzeichnet, dass**
wenigstens ein Ozonsensor (24) vorgesehen ist, der derart ausgebildet ist und derart in dem Fahrzeuginnenraum angeordnet werden kann, dass er einen Ozongehalt im Fahrzeuginnenraum erfasst.

6. Reinigungseinrichtung nach einem der vorhergehenden Ansprüche,
**dadurch gekennzeichnet, dass**
wenigstens ein Personensensor (20) vorgesehen ist, der derart ausgebildet ist und derart in dem Fahrzeuginnenraum angeordnet werden kann, dass er die Anwesenheit von Personen in dem Fahrzeuginnenraum erfasst.

7. Reinigungseinrichtung nach einem der vorhergehenden Ansprüche,
**dadurch gekennzeichnet, dass**
wenigstens eine Antriebsvorrichtung (26) zum Bewegen wenigstens einer der wenigstens einen Strahlungsvorrichtung (10) entlang eines vorbestimmten Weges in dem Fahrzeuginnenraum vorgesehen ist.

8. Reinigungseinrichtung nach einem der vorhergehenden Ansprüche,
**dadurch gekennzeichnet, dass**
wenigstens einer der wenigstens einen Strahlungsvorrichtung (10) in eine Beleuchtungseinrichtung des Fahrzeuginnenraums integriert ist oder mit einer solchen kombiniert ist.

9. Reinigungseinrichtung nach einem der vorhergehenden Ansprüche,
**dadurch gekennzeichnet, dass**
sie ferner wenigstens eine Belüftungseinrichtung (28) zum Belüften des Fahrzeuginnenraums aufweist oder mit einer solchen verbunden ist.

10. Reinigungseinrichtung nach einem der vorhergehenden Ansprüche,
**dadurch gekennzeichnet, dass**
sie ferner wenigstens eine Reinigungsmittel-Ausbringeinrichtung zum Ausbringen eines Reinigungsmittels in den Fahrzeuginnenraum und/oder auf Einrichtungsgegenstände (14) in dem Fahrzeuginnenraum aufweist oder mit einer solchen verbunden ist.

11. Fahrzeug, insbesondere Schienenfahrzeug, mit wenigstens einer Reinigungseinrichtung nach einem der Ansprüche 1 bis 10.

12. Verfahren zum Reinigen von Fahrzeuginnenräumen, insbesondere von Schienenfahrzeugen, bei welchem Einrichtungsgegenstände (14) des Fahrzeuginnenraums und/oder die Luft im Fahrzeuginnenraum (22) mittels wenigstens einer in dem Fahrzeuginnenraum montierten Strahlungsvorrichtung (10) mit einer Strahlung in wenigstens einem vorbestimmten Wellenlängenbereich zwischen etwa 150 nm und etwa 350 nm bestrahlt werden.

13. Verfahren nach Anspruch 12,
**dadurch gekennzeichnet, dass**
eine Bestrahlung in einem ersten vorbestimmten Wellenlängenbereich zwischen etwa 200 nm und etwa 350 nm und/oder eine Bestrahlung in einem zweiten vorbestimmten Wellenlängenbereich zwischen etwa 150 nm und etwa 200 nm durchgeführt werden.

14. Verfahren nach Anspruch 12 oder 13,
**dadurch gekennzeichnet, dass**
zur Ausführung des Reinigungsverfahrens eine Reinigungseinrichtung nach einem der Ansprüche 1 bis 10 verwendet wird.
